(19) Europäisches Patentamt
European Patent Office
Office européen des brevets

(11) **EP 1 307 740 B1**

(12) **EUROPÄISCHE PATENTSCHRIFT**

(45) Veröffentlichungstag und Bekanntmachung des Hinweises auf die Patenterteilung:
**15.06.2011 Patentblatt 2011/24**

(21) Anmeldenummer: **01962620.9**

(22) Anmeldetag: **09.08.2001**

(51) Int Cl.:
**G01N 33/50** $^{(2006.01)}$     **G01N 33/566** $^{(2006.01)}$

(86) Internationale Anmeldenummer:
**PCT/DE2001/003041**

(87) Internationale Veröffentlichungsnummer:
**WO 2002/012885 (14.02.2002 Gazette 2002/07)**

(54) **EINFACHE METHODEN ZUM DRUG MONITORING VON GPIIB/IIIA-REZEPTOR-ANTAGONISTEN**

SIMPLE METHODS FOR THE DRUG MONITORING OF GPIIB/IIIA-RECEPTOR ANTAGONISTS

PROCEDES SIMPLES DE PHARMACOVIGILANCE D'ANTAGONISTES RECEPTEURS GPIIB/IIIA

(84) Benannte Vertragsstaaten:
**AT BE CH CY DE DK ES FI FR GB GR IE IT LI LU MC NL PT SE TR**

(30) Priorität: **09.08.2000 DE 10038900**

(43) Veröffentlichungstag der Anmeldung:
**07.05.2003 Patentblatt 2003/19**

(73) Patentinhaber: **JenAffin GmbH**
**07745 Jena (DE)**

(72) Erfinder:
• **NOWAK, Götz**
**99097 Erfurt (DE)**
• **BUCHA, Elke**
**99094 Erfurt (DE)**

(74) Vertreter: **Vossius & Partner**
**Siebertstrasse 4**
**81675 München (DE)**

(56) Entgegenhaltungen:
**WO-A-97/18474     US-A- 5 763 199**
**US-A- 5 773 228**

• **DATABASE BIOSIS [Online] BIOSCIENCES INFORMATION SERVICE, PHILADELPHIA, PA, US; FURLAN M ET AL: "Exposure of platelet binding sites in von Willebrand factor by absorption onto polystyrene latex particles" Database accession no. 1987:292219 XP002218030 & BIOCHIM BIOPHYS ACTA, Bd. 924, Nr. 1, 1987, Seiten 27-37,**

• **SMITH J.W. ET AL.: "Rapid platelet-function assay. An automated and quantitative cartridge-based method." CIRCULATION, Bd. 99, 2. Februar 1999 (1999-02-02), Seiten 620-625,**

**Beschreibung**

Die Thrombogenität von arteriellen Verschlußkrankheiten und deren Prädisposition wird durch pathologische Plättchenreaktionen bestimmt. Die Blutplättchen-bedingte Startreaktion für manifeste arterielle Makro- und Mikrothrombosen ist Fokus für eine Reihe von Arzneientwicklungen. In den letzten Jahren hat sich das therapeutische Prinzip der Blockade von GPIIb/IIIa-Rezeptoren der Plättchen als besonders aussichtsreich erwiesen. Es konnte in verschiedenen Studien (Capture, Epilog, Epic, Epistent u.a.) zweifelsfrei gezeigt werden, daß sich durch Substanzen, die die Fibrinogenrezeptoren der Plättchen (GPIIb/IIIa) blockieren, eine thrombotische Reaktion verhindern bzw. antagonisieren läßt. Die größten Erfahrungen sind bisher mit einem monoklonalen Antikörperfragment, dem sogenannten Abciximab (Reopro), gesammelt worden, aber auch weitere Substanzen aus anderen Quellen, wie natürliche Disintegrine oder Peptidantagonisten bzw. Peptidomimetika, befinden sich augenblicklich in der klinischen Prüfung.

Das wesentliche Problem in der Praxis mit diesen Substanzen besteht darin, daß sie erst dann eine klinische Effektivität aufweisen, wenn 60-80% der GPIIb/IIIa-Rezeptoren aller in der Zirkulation befindlicher Plättchen blockiert sind. Die äußerst geringe therapeutische Breite dieser Substanzen ist damit zu begründen, daß bereits bei 90-95% Rezeptorblockade eine exzessive Blutungsneigung besteht. Diese schwere Nebenwirkung limitiert auch eine breite Anwendung dieser neuen Substanzgruppe.

In der Vergangenheit hat es nicht an Versuchen gefehlt, die Kontrolle des Blutspiegels mit Hilfe von verschiedensten plättchenfunktionserfassenden Methoden zu erreichen. Eine Korrelation zwischen Blutspiegel der GPIIb/IIIa-Antagonisten und der Wirkung ist indes nicht gegeben, da keine direkte Beziehung zwischen Höhe des Substanzblutspiegels und der Stärke der Aggregationshemmung vorhanden ist. Bedingt wird diese fehlende Korrelation vor allem durch die variable Zahl der zirkulierenden Blutplättchen, aber auch die Zahl der an der Plättchenoberfläche exponierten Integrinmoleküle. Zum Wirkungsnachweis werden am häufigsten Methoden der Plättchenaggregationsmessung eingesetzt. Die im plättchenhaltigen bzw. plättchenreichen Plasma durchgeführten Aggregationsagonistenreaktionen auf Kollagen oder ADP sind zeit- und kostenaufwendig und geben nur indirekt die tatsächlichen Verhältnisse im Patientenblut wieder, da sie aufgrund der unumgänglichen Separation und Aufreicherung des Patientenplasmas mit Blutplättchen keine naturidentischen Reaktionen zulassen.

Eine POCT-fähige Vollblutmethode der Plättchenaggregation, die Ultegra-Accumetics-Methode, ist im therapeutisch-toxischen Bereich der GPIIb/IIIa-Rezeptorantagonisten zu stark streuend und extrem kostenintensiv. Auch sie benutzt einen starken Aggregationsauslöser als Meßprinzip. Weiterhin sind Untersuchungen an Gerinnungsglobaltests, wie Thrombelastographen und Prothrombinzeit zu stark varrierend, so daß eine Zuordnung der prozentualen Hemmhöhe der Plättchenfunktion zu diesen Tests nahezu aussichtslos ist.

Es ist somit die Aufgabe der Erfindung, die vorstehend genannten Nachteile des Standes der Technik zu überwinden. Gelöst wurde diese Aufgabe durch den überraschenden Befund, daß spezielle Polymeroberflächen für Blutplättchen eine optimale Adhäsionsfläche darstellen.

Die bisher bekannten Plättchenadhäsions- bzw. Plättchenausbreitungstests sind Mikroskopie-benützende qualitative Methoden, die auf Glas-Slides stattfinden. Die hier vorgestellte Methode benutzt glasähnliche Polymere, die zu speziellen Mikropartikeln mit "rauhen" Oberflächen geformt wurden. Diese Mikropartikeloberflächen sind die direkten Adhäsionspartner für aktivierte Plättchen. Durch die Zugabe von einer definierten Quantität von monodispersen Polymerpartikeln zu antikoaguliertem Vollblut und kurzzeitiger Schüttelinkubation bei Raumtemperatur kann mittels Differenzmessung der Plättchenzahl in der Suspension vor und nach Polymerpartikel-Schüttlung ein Adhäsionsindex bestimmt werden. Die hier vorgestellte Methode ist so ausgelegt, daß bei nicht gerinnungsgestörten Patienten und Probanden dieser Adhäsionsindex 50 beträgt ($50\pm8$). Bei Vorliegen einer Adhäsionshemmung nach Behandlung mit GPIIb/IIIa-Rezeptorantagonisten kommt es zu einer verminderten Adhäsion, die streng dosisabhängig ist, in diesem Falle würde der Adhäsionsindex <40 sein. Zum anderen kann auch dieser Test benutzt werden, um eine erhöhte Plättchenadhäsion bei Patienten zu erfassen.

Die Erfindung betrifft somit die Verwendung von definierten Mengen von monodispersen Partikeln, erhalten aus porösem Polyphenylmethacrylat. Die benutzten Partikel können aus monodispersen Polymerpartikeln mit einem Durchmesser zwischen 1 und 20 $\mu$m bestehen besser zwischen 1 und 10 $\mu$m, am geeignetsten sind monodisperse poröse Partikel in der Größe von 5-6 $\mu$m. Die für die quantitative Adhäsions-Methode benutzten Hilfsrnateriallen sind lediglich ein Kleinschüttelgerät (M51 von IKA) und ein Blutzellzählgerät, wie z.B. das Gerät Celldyn 1600 von Abbott oder ähnliche Blutzellanalysatoren, wie sie in jedem klinisch-chemischen Labor zur Anwendung kommen. Innerhalb von 15 min ist der entsprechende Wert zu erhalten. Die Methode kann direkt am Krankenbett bzw. am Herz-Kreislauf-Funktionsmeßplatz durchgeführt werden. Die Methode hat POCT-Qualität, sie ist nicht von der Zahl der zirkulierenden Plättchen (80-40000 Plättchen/$\mu$l) abhängig, sie wird auch nicht durch variable Fibrinogenspiegel (1-5 g/l) beeinflußt.

Rezeptur: 0,2 ml antikoaguliertes Blut z.B. Zitratblut (1/10 v/v)

+ 0,002 ml 5 % Partikelsuspension

- 10 min schütteln bei 600/min

- a) Bestimmung der Plättchenzahl vor Beginn der Schüttelung
- b) Bestimmung der Plättchenzahl nach der Schüttelung
-

$$AI \text{ (Adhäsionsindex)} = \frac{\text{(Plättchenzahl vor Schüttelung)} - \text{(PZ nach Schüttelung)}}{\text{(PZ vor Schüttelung)}} \times 100$$

**Patentansprüche**

1. Verwendung von monodispersen Polymerteilchen aus porösen Polyphenylmethacrylat zur Messung der Adhäsion von Blutplättchen in vitro, wobei die Polymerteilchenoberflächen als direkte Adhäsionspartner für aktivierte Blutplättchen fungieren.

2. Verwendung nach Anspruch 1, wobei die Polymerteilchen einen Durchmesser von 1 bis 20 $\mu$m aufweisen.

3. Verwendung nach Anspruch 1 oder 2, wobei der Durchmesser 1 bis 10 $\mu$m beträgt.

4. Verwendung nach Anspruch 3, wobei der Durchmesser 5 bis 6 $\mu$m beträgt.

5. Verfahren zur in vitro Bestimmung der Blutplättchenadhäsion, wobei entsprechend aufbereitetes Blut mit den Polymerteilchen, wie in einem der Ansprüche 1 bis 4 definiert, geschüttelt und Blutplättchen vor und nach dem Schuttteln mit einem herkömmlichen Blutzellzählgerät bestimmt werden.

6. Verwendung nach einem der Ansprüche 1 bis 4, wobei sie zur schnellen, exakten Diagnostik von erhöhter oder verringerter Adhäsivität von Blutplättchen dient.

7. Verwendung nach einem der Ansprüche 1 bis 4, wobei sie zum schnellen, exakten Monitoring von Plättchenfunktionshemmstoffen, wie GPIIb/IIIa-Rezeptorantagonisten bei Patienten geeignet ist.

**Claims**

1. Use of monodisperse polymer particles of porous polyphenylmethacrylate for measuring the adhesion of platelets in vitro, said polymer particle surfaces acting as direct adhesion partners for activated platelets.

2. Use according to claim 1 wherein the polymer particles exhibit a diameter of 1 to 20 $\mu$m.

3. Use according to claim 1 or 2 wherein the diameter is 1 to 10 $\mu$m.

4. Use according to claim 3 wherein the diameter is 5 to 6 $\mu$m.

5. Method for the in vitro determination of the platelet adhesion, wherein blood prepared accordingly is shaken with the polymer particles, as defined in any one of claims 1 to 4, and platelets are determined before and after the shaking with a conventional blood cell counting device.

6. Use according to any one of claims 1 to 4, wherein it serves the purpose of a fast and exact diagnosis of increased or reduced adhesivity of platelets.

7. Use according to any one of claims 1 to 4, wherein it is suitable for fast and exact monitoring of substances inhibiting platelet function in patients, such as GPIIb/IIIa receptor antagonists.

**Revendications**

1.  Utilisation de particules polymériques monodispersées constituées de méthacrylate de polyphényle poreux pour mesurer l'adhésion des plaquettes sanguines in vitro, dans laquelle les surfaces des particules polymériques font office de partenaires d'adhésion directs pour les plaquettes sanguines activées.

2.  Utilisation selon la revendication 1, dans laquelle les particules polymériques présentent un diamètre de 1 à 20 $\mu$m.

3.  Utilisation selon la revendication 1 ou 2, dans laquelle le diamètre est de 1 à 10 $\mu$m.

4.  Utilisation selon la revendication 3, dans laquelle le diamètre est de 5 à 6 $\mu$m.

5.  Procédé de détermination in vitro de l'adhésion des plaquettes sanguines, dans lequel du sang préparé de manière appropriée est agité avec les particules polymériques, telles que définies dans l'une des revendications 1 à 4, et les plaquettes sanguines sont déterminées avant et après agitation avec un appareil traditionnel de comptage du nombre de cellules sanguines.

6.  Utilisation selon une des revendications 1 à 4, qui sert à un diagnostic rapide et exact de l'adhésivité augmentée ou réduite des plaquettes sanguines.

7.  Utilisation selon une des revendications 1 à 4, qui est appropriée au suivi rapide et exacte des inhibiteurs de la fonction des plaquettes, comme les antagonistes du récepteur GPIIb/IIIa, chez un patient.